# EUROPEAN PATENT SPECIFICATION

(11) **EP 2 783 719 B1**
(45) Date of publication and mention of the grant of the patent: **02.05.2018**
(21) Application number: 14160687.1
(22) Date of filing: 19.03.2014
(51) Int. Cl.: A61M 5/14, A61M 5/162, A61J 1/20, A61J 1/16

(54) **Vented Luer cone connector**
Belüfteter Luer-Konus-Verbinder
Connecteur d'embout Luer ventilé

(30) Priority: 20.03.2013 US 201313847663
(43) Date of publication of application: 01.10.2014
(73) Proprietor: Caesarea Medical Electronics Ltd., 30889 Caesarea (IL)
(72) Inventor: Barak, Swi, 30889 Caesaria (IL)
(74) Representative: Harrison IP Limited

(56) References cited:
- EP-A2- 0 169 722
- WO-A2-2008/129550
- US-A- 4 623 343
- US-A1- 2011 049 866

## Description

### Field Of The Invention

The present invention relates to the field of medical devices, and more particularly to the field of vented connectors to be used with male Luer fittings.

### Background Of The Invention

Needleless connectors and Luer fittings are known in the art. Luer fittings are conical fittings having a 6% taper that are typically used for syringes, needles, and certain other medical equipment.

US Patent No. 7,497,848 to Leinsing et al. discloses a needleless connector for medical use, adapted to facilitate the flow of fluid therethrough. The connector includes a housing having an inlet port and an outlet port. The connector also includes a flex-tube assembly defining a fluid path between the inlet port and the outlet port. The flex-tube assembly is movable between uncompressed and compressed states. The flex-tube assembly has a first internal volume when in the uncompressed state and a second internal volume, greater than or substantially equal to the first internal volume, when in the compressed state.

US Patent No. 7,184,825 to Leinsing et al. discloses a needleless connector similar to the connector of '848.

US Patent No. 6,706,022 to Leinsing et al. discloses a needleless connector similar to the connector of '848.

US Patent Application No. 2009/0149819 to Chelak discloses a device for reducing microbial contamination. The device includes a housing including a proximal portion defining a cavity containing a quantity of microbial reducing agent, a distal portion defining a cavity for selective coupling with the connector of the indwelling line, and a partition separating the proximal and distal cavities, wherein the proximal and distal cavities are in fluid communication with one another; and a deformable member overlying an end of the proximal portion for maintaining the microbial reducing agent within the proximal cavity thereof. The microbial reducing agent being dispensable into the distal cavity, at least when the medical device is connected to the connector of the indwelling line and reduce microbes present thereon.

US Patent Application No. 2007/0007478 to Leinsing at el. discloses a needle free medical connector that includes a housing with a first port and a second port. The connector also includes a piston element defining a fluid passageway between the first and second ports. The piston element is movable between flow and non-flow positions. The piston element has a compressible section having a variable inner width that forms a part of the flow path through the connector. As the piston is compressed to the flow position, the compressible section self-expands in width thereby maintaining or increasing the volume of the fluid passageway through the connector. The compressible section has a configuration permitting the continuous flow of fluid through its entirety.

US Patent No. 4,623,343 to Thompson discloses a connector that provides an air vent extending into a syringe barrel in order to prevent bubbles from being drawn out of the syringe outlet. Despite the efficiency of the connector, it is relatively complicated, comprises several parts, and requires assembly of the various parts in order to use the connector.

EP 0 169 722 to Greenland discloses a similar connector, also having a ball valve and a cannula which extends into the fluid. Similar to the connector of '343, this connector is relatively complicated, comprises several parts, and requires assembly of the various parts in order to use the connector.

WO 2008/129550 to Kriheli discloses an apparatus that allows contamination-free transfer of a liquid from one container to another. This apparatus suffers from the same disadvantages described in the two previous paragraphs.

### Summary Of The Invention

With a view to mitigating at least some of the foregoing disadvantages of the prior art, the present invention provides a vented connector as hereinafter set forth in Claim 1 of the appended claims.

Features of embodiments of the invention are hereinafter set forth in the dependent claims.

### Brief Description Of The Drawings

The invention will now be described further, by way of example, with reference to the accompanying drawings, in which:
Fig. 1 is a perspective view of a vented connector according to a first embodiment of the present invention;
Fig. 2 is a cross-sectional view of the vented connector of Fig. 1;
Fig. 3 is a perspective view of a casing, preferably to be used with the vented connector, according to another embodiment of the present invention; and
Fig. 4 is an exploded perspective view of the casing of Fig. 3.

### Description Of Preferred Embodiments

Illustrative embodiments of the invention are described below. In the interest of clarity, not all features of an actual implementation are described within the present specification. It could be appreciated that during the development of any actual embodiment, numerous implementation-specific decisions must be made in order to achieve developer's specific goals, such as compliance with technology or business-related constraints, which may vary from one implementation to another. Moreover, it could be appreciated that the effort of such a development might be complex and time-consuming, but would nevertheless be a routine undertaking for those of ordinary skill in the art having the benefit of this disclosure.

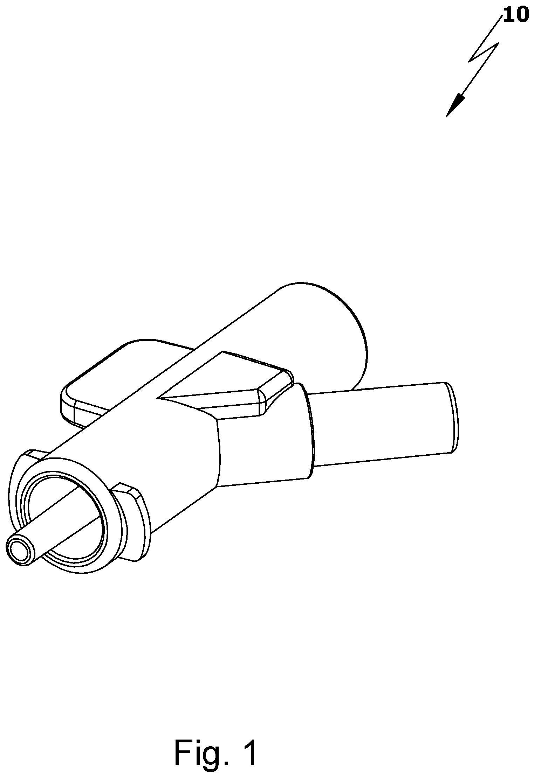

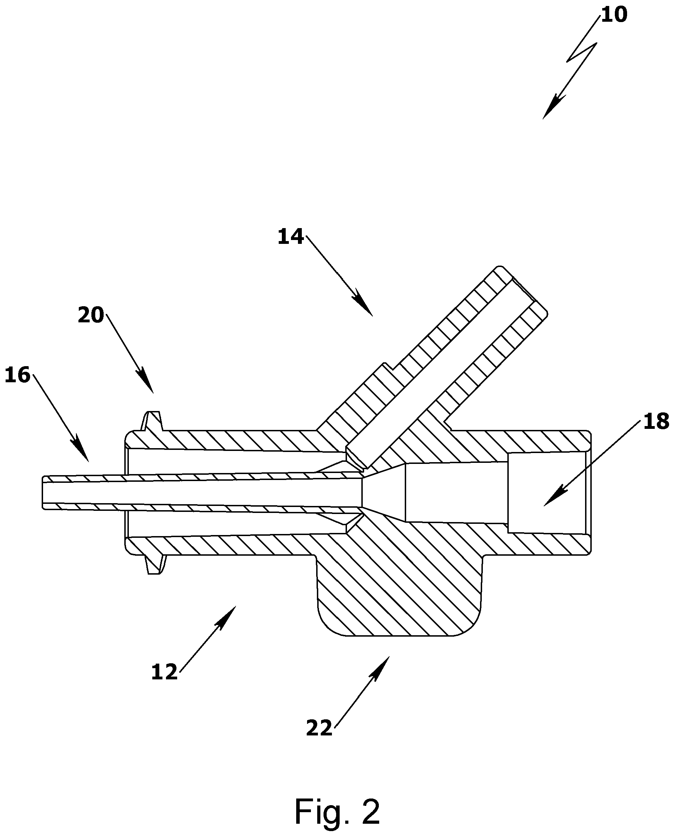

Figs. 1 and 2 show a vented connector 10 according to the present invention. The connector 10 comprises an axial portion 12 and bifurcating portion 14 branching off at an angle from the axial portion 12. The axial and bifurcating portion 12 and 14, as best shown in Figure 2, are formed in one piece. The axial portion 12 comprises an interior post portion 16, an air inlet 18 and an exterior housing portion 20. The lumen of the interior post portion 16 forms a continuum with the air inlet 18. The post portion 16 is located at a proximal end of the connector 10, and the air inlet 18 is located at a distal end of the connector 10. The exterior housing portion 20 may be furnished with a male Luer lock matching protrusions/jags, and/or a gripping handle or lever 22.

The housing portion 20 is adapted to receive a male Luer tip fitting of a receptacle, preferably of a syringe (not shown), so that the exterior surface of the male Luer fitting abuts the interior surface of the exterior housing portion 20 in a sealing engagement. The lumen of the exterior housing portion 20 forms a continuum with a lumen of the bifurcating portion 14, which serves as the outlet for the fluid outflow from the exterior housing portion 20 of the connector 10, as will be described below. The bifurcating portion 14 preferably matches standard male/female Luer fittings and may be furnished with corresponding male/female Luer lock protrusions, jags, grooves or ridges.

The outer diameter of the post portion 16 is somewhat smaller than the inner diameter of the male Luer tip fitting (not shown) of the receptacle (not shown) inserted into the exterior housing portion 20, thus providing a fluid flow in-between the exterior surface of post portion 16 and the interior surface of the male Lure tip fitting.

In order to facilitate the co-occurrence of the air flow from the air inlet 18 (hereinafter will be referred to as "inlet 18") throughout the post portion 16 into the interior of the receptacle and the fluid flow from the interior of the receptacle in-between the exterior surface of post portion 16 (hereinafter will be referred as "post 16") and the interior surface of the male Luer tip fitting into the lumen of the exterior housing portion 20 (hereinafter will be referred to as "housing 20") without intermingling with each other, the assembly comprising the receptacle/male Luer tip fitting and the connector 10 is to assume an upright position, i.e., so that the distal end of the connector 10 faces downwards, while the proximal end of the connector 10 faces upwards.

The post 16 preferably surpasses the male Luer tip fitting when the latter is inserted into the former, thereby, the air that is drawn into the connector 10 via the inlet 18 passes throughout the post 16 into the interior lumen of the receptacle, without intermingling with the concomitantly occurring fluid flow from the lumen of the receptacle into the lumen of the housing 20, in-between the exterior surface of the post 16 and the interior surface of the male Luer tip fitting.

The post 16 preferably surpasses the male tip fitting, so that the entrance point of the air drawn into the receptacle, namely the proximal end of the post 16, is located above the outlet at the base of male Luer tip fitting, namely where the fluid is drawn from the receptacle in-between the exterior surface of the post 16 and the interior surface of the male Luer tip fitting.

In accordance with some embodiments of the connector, the tolerance of the gap between the proximal end of the post 16 and the aforementioned outlet at the base of the male Luer tip fitting, namely where the fluid is drawn from the receptacle, is more than about 2 mm but less than about 6 mm.

The inlet 18 is furnished with a first type a specific filter or membrane element that selectively allows for the flow of air to pass therethrough into the lumen of the post 16, while preventing an outflow of fluid therethrough, from the lumen of the post 16 out of the inlet 18. The filter or membrane element of the first type sustains the flow of air therethrough while being wet, soaked, drenched or otherwise saturated with the fluid, which may gutter thereto from the receptacle due to gravitational force.

The selective filter or membrane element of the first type preferably delivers a sterile air/gas into the connector 10. Examples of the filter or membrane element of the first type include Versapor (RTM) R membrane - FluoRepel (TM) Treated membrane, available from Pall Corp corporation, 2200 Northern Boulevard East Hills, NY 11548-1289, Cat. Nos. 6694197, 6704192, and 6304185.

The bifurcating portion 14, which serves as the outlet for the fluid outflow from the housing 20 of the connector 10, can be furnished with a second type of filter or a membrane element functioning in essentially opposite manner to the filter or membrane element of the first type. The specific filter or membrane element of the second type is permeable to fluids, selectively allows for the outflow of fluids to pass therethrough, into a tubing set that may be connected thereto, while retaining the air and preventing an outflow thereof from the connector 10, thereby precluding the formation of air emboli in the fluid drained from the connector 10.

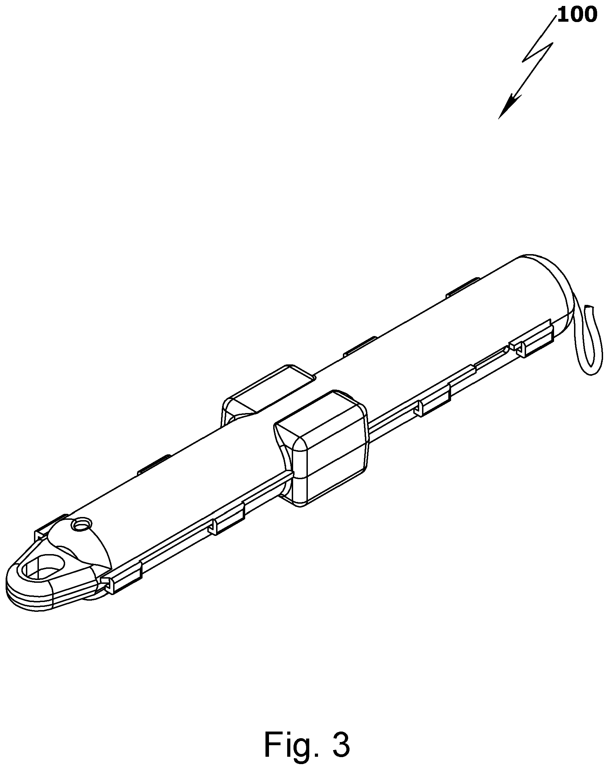

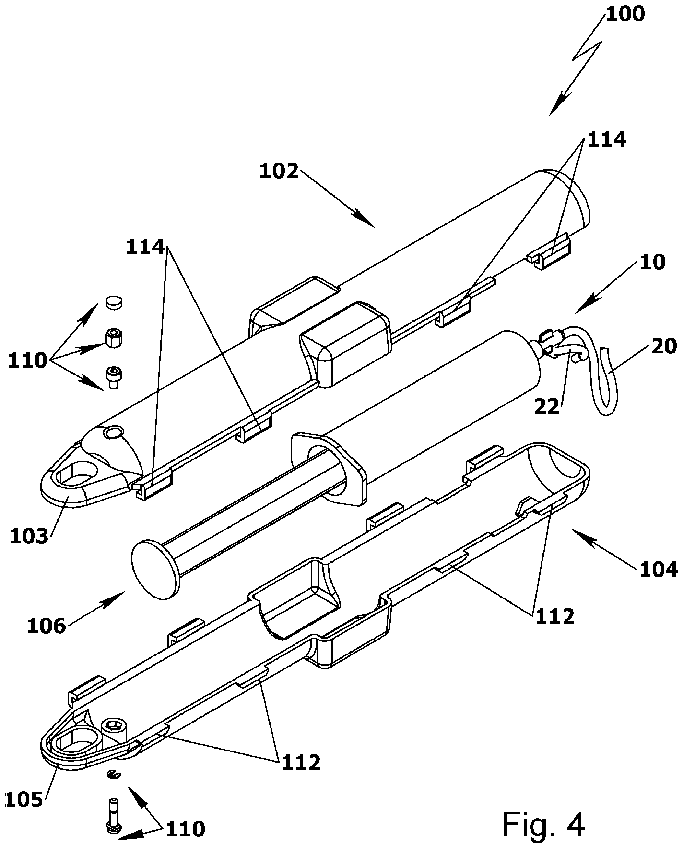

In order to achieve the upright positioning of the aforementioned assembly comprising the receptacle/male Luer tip fitting and the vented connector 10 shown in Fig. 1 and 2, i.e., that the distal end of the connector 10 faces downwards while the proximal end of the connector 10 faces upwards, the connector 10 is preferably incorporated within the assembly set up of the system 100 shown in Figs. 3 and 4, to which reference is now made.

The system 100 comprises a first casing subunit 102 and a second casing subunit 104. The first and second subunits 102 and 104 forming the casing, and having respective eyelet portions 103 and 105. The casing subunits 102 and 104 are adapted to accommodate a receptacle 106. The receptacle 106 is typically a syringe of a standard type. In an assembled state, the subunits 102 and 104 may be secured to each other by means of a bolt sub-assembly 110. The bolt sub-assembly 110 may include, in a non-limiting manner, bolts, nuts, washers, split washers or spring washers or rings, and a cork element. Additionally or alternatively, in the assembled state, the subunits 102 and 104 may be secured to each other by means of slidably interlocking elements 114 and 112, respectively.

The connector 10 is preferably provided within a sterilized packaging (not shown). The connector 10 is mounted onto the male Luer tip fitting of the syringe receptacle 106. The system 100 is preferably employed with infusion sets, pumps, connectors or manifolds and used to administrate the content of the receptacle, or particularly of a syringe receptacle, into the patient or into a perfusion tubing system, exemplified by a conduit 20, which may be connected to the air inlet of the connector 10, and a conduit 22, which is connected to the fluid outlet, i.e., the bifurcating portion of the connector 10.

The system 100 is typically suspended in an upright position from the eyelet tab and enables administrating the content of a rigid-shell receptacle, such as the syringe 106, by maintaining an inflow of air thereto, as elaborated supra. The rigid-shell receptacle, such as the syringe 106, may contain any medical fluid, inter alia, including saline or other physiological aqueous solutions, liquid or dissolved drugs or medications, etc.

It should be noted that directional terms appearing throughout the specification and claims, e.g. "forward", "rear", "upper", "lower" etc., are used as terms of convenience to distinguish the location of various surfaces relative to each other. These terms are defined with reference to the figures, however, they are used for illustrative purposes only, and are not intended to limit the scope of the appended claims.

Although the present invention has been described to a certain degree of particularity, it should be understood that various alterations and modifications could be made without departing from the invention as set out in the appended claims.

For example, the components shown in the drawing are not necessarily shown in scale with relation to each other, however, it should be provided that they follow the principles disclosed and claimed.

## Claims

1. A vented connector (10) to be used with a male Luer tip fitting of a receptacle to permit liquid to be withdrawn from the receptacle and air to be admitted into the receptacle without intermingling between the admitted air and the withdrawn liquid, wherein the connector comprises an axial portion (12) and a bifurcation portion (14) formed together in one piece; the axial portion (12) comprises an exterior tubular housing (20) and an interior tubular post (16) surrounded by and spaced from the exterior tubular housing (20), the post having a proximal end extending axially beyond the exterior tubular housing, the interior post (16) has a lumen for admission of air into the receptacle in fluid flow communication with an air inlet (18) located in line with the axis of the interior post, an annular passage is defined, during use, in-between an exterior surface of the post (16) and an interior surface of said male Luer tip fitting into which the connector is inserted, the passage being in fluid communication with a lumen in the bifurcation portion to permit flow of liquid out of the receptacle to a liquid outlet defined by the bifurcation portion, and a filter or a membrane element is provided in the air inlet to allow a flow of air to pass therethrough while preventing an outflow of liquid therefrom.

2. A vented connector as claimed in claim 1, wherein the proximal end of the post (16) extends axially beyond the exterior tubular housing (20) by more than 2mm and less than 6mm.

3. A vented connector as claimed in claim 1 or 2, wherein the bifurcating portion (14) matches standard male/female Luer fittings.

4. A vented connector as claimed in claim 3, wherein the bifurcating portion (14) is provided with corresponding male/female Luer lock protrusions, jags, grooves or ridges.

5. A vented connector as claimed in any preceding claim, wherein the bifurcating portion (14) is fitted with a filter or a membrane element that is liquid permeable but prevents through flow of air.

## Patentansprüche

1. Belüfteter Verbinder (10) zur Verwendung mit einem männlichen Luer-Spitzenanschluss eines Behältnisses, um die Entnahme von Flüssigkeit aus dem Behältnis und das Hereinlassen von Luft in das Behältnis zu ermöglichen, ohne dass die hereingelassene Luft und die entnommene Flüssigkeit vermischt werden, wobei der Verbinder einen axialen Abschnitt (12) und einen Gabelungsabschnitt (14) umfasst, die gemeinsam in einem Stück ausgebildet sind; wobei der axiale Abschnitt (12) ein äußeres röhrenförmiges Gehäuse (20) und einen inneren röhrenförmigen Pfosten (16) umfasst, der von dem äußeren röhrenförmigen Gehäuse (20) umgeben und zu diesem beabstandet ist, wobei der Pfosten ein proximales Ende aufweist, das sich axial über das äußere röhrenförmige Gehäuse hinaus erstreckt, wobei der innere Pfosten (16) ein Lumen zum Hereinlassen von Luft in das Behältnis in Fluidströmungskommunikation mit einem Lufteinlass (18) aufweist, ausgerichtet mit der Achse des inneren Pfostens, wobei während der Verwendung ein ringförmiger Durchgang zwischen einer äußeren Oberfläche des Pfostens (16) und einer inneren Oberfläche des männlichen Luer-Spitzenanschlusses definiert ist, in welchen der Verbinder eingeführt wird, wobei sich der Durchgang in Fluidkommunikation mit einem Lumen in dem Gabelungsabschnitt befindet, um einen Flüssigkeitsfluss aus dem Behältnis zu einem Flüssigkeitsauslass zu ermöglichen, der durch den Gabelungsabschnitt definiert ist, und wobei ein Filter oder ein Membranelement in dem Lufteinlass bereitgestellt ist, um es zu ermöglichen, dass eine Luftströmung dort hindurch verläuft, während es verhindert wird, dass Flüssigkeit von dort ausströmt.

2. Belüfteter Verbinder nach Anspruch 1, wobei sich das proximale Ende des Pfostens (16) axial um mehr als 2 mm und weniger als 6 mm über das äußere röhrenförmige Gehäuse (20) hinaus erstreckt.

3. Belüfteter Verbinder nach Anspruch 1 oder 2, wobei der Gabelungsabschnitt (14) mit genormten männlichen/weiblichen Luer-Anschlüssen zusammenpasst.

4. Belüfteter Verbinder nach Anspruch 3, wobei der Gabelungsabschnitt (14) mit entsprechenden Vorsprüngen, Zacken, Rillen oder Rippen eines männlichen/weiblichen Luer-Locks bereitgestellt ist.

5. Belüfteter Verbinder nach einem der vorstehenden Ansprüche, wobei der Gabelungsabschnitt (14) mit einem Filter oder einem Membranelement ausgestattet ist, das für Flüssigkeit durchlässig ist, jedoch das Hindurchströmen von Luft verhindert.

## Revendications

1. Connecteur ventilé (10) destiné à être utilisé avec un raccord d'extrémité Luer mâle d'un récipient pour permettre le retrait d'un liquide du récipient et l'admission d'air dans le récipient sans mélange entre l'air admis et le liquide retiré, le connecteur comprenant une partie axiale (12) et une partie de bifurcation (14) formées ensemble en une seule pièce ; la partie axiale (12) comprenant un boîtier tubulaire extérieur (20) et un montant tubulaire intérieur (16) entouré par et espacé du boîtier tubulaire extérieur (20), le montant ayant une extrémité proximale s'étendant axialement au-delà du boîtier tubulaire extérieur, le montant intérieur (16) ayant une lumière pour l'admission d'air dans le récipient en communication fluidique avec une entrée d'air (18) située en ligne avec l'axe du montant intérieur, un passage annulaire étant défini, pendant l'utilisation, entre une surface extérieure du montant (16) et une surface intérieure dudit raccord d'extrémité Luer mâle dans lequel le connecteur est inséré, le passage étant en communication fluidique avec une lumière dans la partie de bifurcation pour permettre l'écoulement de liquide hors du récipient vers une sortie de liquide définie par la partie de bifurcation, et un filtre ou un élément à membrane étant disposé dans l'entrée d'air pour permettre à un flux d'air de passer au travers tout en empêchant le liquide d'en sortir.

2. Connecteur ventilé selon la revendication 1, l'extrémité proximale du montant (16) s'étendant axialement au-delà du boîtier tubulaire extérieur (20) de plus de 2 mm et de moins de 6 mm.

3. Connecteur ventilé selon la revendication 1 ou 2, la partie de bifurcation (14) correspondant à des raccords Luer mâle/femelle standard.

4. Connecteur ventilé selon la revendication 3, la partie de bifurcation (14) disposant de saillies, dentures, rainures ou arêtes de verrouillage Luer mâle/femelle correspondantes.

5. Connecteur ventilé selon l'une quelconque des revendications précédentes, la partie de bifurcation (14) comprenant un filtre ou un élément à membrane qui est perméable aux liquides mais empêche le passage de l'air.
